# FASCICULE DE BREVET EUROPEEN

(11) **EP 0 920 626 B1**
(45) Date de publication et mention de la délivrance du brevet: **26.02.2003**
(21) Numéro de dépôt: 98932247.4
(22) Date de dépôt: 19.06.1998
(51) Int. Cl.: G01N 33/543, C07K 17/00, G01N 33/532

(54) **PROCEDE DE MISE EN EVIDENCE D'UN MATERIEL BIOLOGIQUE CIBLE, PHASE DE CAPTURE, PHASE DE DETECTION ET REACTIF LES CONTENANT**
VERFAHREN ZUR DETEKTIERUNG EINES BIOLOGISCHEN MATERIALS, EINGANGPHASE, DETEKTIONSPHASE UND DIESE ENTHALTENDE REAGENZ
METHOD FOR ISOLATING A TARGET BIOLOGICAL MATERIAL, CAPTURE PHASE, DETECTING PHASE AND REAGENT CONTAINING THEM

(30) Priorité: 20.06.1997 FR 9708055
(43) Date de publication de la demande: 09.06.1999
(73) Titulaire: BIO MERIEUX, 69280 Marcy l'Etoile (FR)
(72) Inventeur: MALLET, François, F-69100 Villeurbanne (FR); DELAIR, Thierry, F-69700 Echalas (FR); LADAVIERE, Catherine, F-69110 Sainte Foy lès Lyon (FR); NOVELLI-ROUSSEAU, Armelle, F-38180 Seyssins (FR); CHARLES, Marie-Hélène, F-69420 Condrieu (FR)
(74) Mandataire: Guerre, Dominique
(86) Numéro de dépôt international: FR9801299
(87) Numéro de publication internationale: WO98059241

(56) Documents cités:
- EP-A- 0 319 012
- EP-A- 0 561 722
- EP-A- 0 632 269
- WO-A-96/22533
- YANG ET AL: "COVALENT IMMOBLIZATION OF IMMUNOGLOBULIN TO POLYSTYRENE AND SILICON NITRIDE CHIPS" AMERICAN CHEMICAL SOCIETY. ABSTRACTS OF PAPER. AT THE NATIONAL MEETING, vol. 192, 7 septembre 1986, ANAHEIM, CA, page 13 XP000563391

## Description

L'invention concerne un procédé de mise en évidence d'un matériel biologique cible contenu dans un échantillon, utilisant une phase de capture comprenant une molécule organique ayant au moins une fonction réactive, et au moins un matériel protéique susceptible de reconnaître ou de se lier, spécifiquement et directement ou indirectement, au matériel biologique cible, ledit matériel protéique possédant un site spécifique de liaison par covalence à la fonction réactive de la molécule organique.

On connaît selon le document EP-A-0 319 012 un procédé d'immobilisation d'une protéine sur un support solide, par l'intermédiaire de fonctions réactives, dans le but de stimuler la prolifération ou le développement de cellules T et la production de lymphocytes tueurs. A cet effet, la protéine présente, sur son extrémité C-terminale, un site de liaison par covalence pour se lier auxdites fonctions réactives, qui consiste en une suite d'acides aminés dans laquelle sont répartis deux à quatre résidus lysine.

Le problème posé par le site de liaison par covalence de cet art antérieur, réside dans le fait que, dans une application diagnostique, les résultats de mise en évidence d'un matériel biologique cible avec lequel il est susceptible de se lier, sont similaires à ceux obtenus avec un matériel protéique non doté d'un tel site de liaison.

Selon l'invention, on apporte un procédé de mise en évidence d'un matériel biologique cible en utilisant au moins une phase de capture qui comprend un matériel protéique possédant un site de liaison par covalence qui permet une orientation efficace dudit matériel protéique, et conduit à une détection sensible et performante dudit matériel biologique.

Ainsi le procédé de l'invention, pour mettre en évidence un matériel biologique cible contenu dans un échantillon, comprend les étapes suivantes :
on dispose d'une phase de capture qui comprend une molécule organique ayant au moins une fonction réactive et au moins un matériel protéique susceptible de reconnaître ou de se lier, spécifiquement et directement ou indirectement, au matériel biologique cible, ledit matériel protéique possédant un site spécifique de liaison par covalence à la fonction réactive de la molécule organique, qui consiste en au moins un tag comprenant au moins six résidus lysine, ou dérivé de lysine, contigus,
on met en contact ledit matériel biologique cible avec au moins la phase de capture, et
on détecte le matériel biologique cible fixé sur la phase de capture.

Par mise en évidence d'un matériel biologique cible selon l'invention, on comprend la fixation, la séparation, l'isolement, la détection et/ou quantification de ce matériel, l'enrichissement d'une fraction en matériel biologique cible, selon une méthode de fixation spécifique ou aspécifique, de manière qualitative et/ou quantitative.

Selon une variante du procédé, la phase de capture peut au surplus comprendre un marqueur, et dans ce cas notamment, elle peut consister en une phase de détection.

Selon une autre variante du procédé, on dispose en outre d'une phase de détection qui comprend une molécule organique ayant au moins une fonction réactive, au moins un matériel protéique susceptible de reconnaître ou de se lier, spécifiquement et directement ou indirectement, au matériel biologique cible, et un marqueur, ledit matériel protéique possédant un site spécifique de liaison par covalence à la fonction réactive de la molécule organique, qui consiste en au moins un tag comprenant au moins six résidus lysine, ou dérivé de lysine, contigus.

Dans ce cas, les molécules organiques de la phase de capture et de la phase de détection, respectivement, peuvent être identiques ou différentes, et les matériels protéiques de la phase de capture et de la phase de détection, respectivement, peuvent être identiques ou différents.

Un échantillon tel qu'on l'entend selon l'invention, comprend tout échantillon susceptible de contenir un matériel biologique, notamment un échantillon tel que celui obtenu à partir d'un fluide biologique, un échantillon d'origine alimentaire, ou une culture cellulaire.

L'échantillon consiste en tout ou partie d'un autre échantillon, en particulier il peut consister en un aliquote, une dilution.

Un matériel protéique selon l'invention comprend les protéines, en particulier protéines recombinantes, notamment antigènes, anticorps, les peptides tels que peptides de synthèse. Le procédé de l'invention pourrait aussi être mis en oeuvre avec un matériel comme les analogues peptides d'acides nucléiques (PNA).

Par molécule organique, on entend une molécule de taille variable ; ainsi on comprend aussi bien une petite molécule telle qu'un haptène, qu'une macromolécule comme un polymère.

A titre d'exemple d'un haptène, on peut citer une hormone, une vitamine, telle que la biotine ou un médicament. Dans ce cas, le procédé de l'invention peut comprendre, avant l'étape de détection du matériel biologique cible, une étape de fixation de la molécule organique sur une molécule porteuse. Préférentiellement, l'haptène est la biotine et la molécule porteuse est l'avidine.

Un polymère tel qu'employé selon l'invention est un polymère sous forme particulaire ou linéaire. Il peut s'agir d'un homopolymère notamment choisi parmi la polylysine et la polytyrosine, ou bien d'un copolymère notamment choisi parmi les copolymères d'anhydride maléique, les copolymères de N-vinyl-pyrrolidone, les polysaccharides, naturels ou synthétiques, les polynucléotides et les copolymères d'acides aminés comme les enzymes. Avantageusement, c'est un copolymère choisi parmi le copolymère anhydride maléique / méthyl-vinyl-éther, le copolymère N-vinyl-pyrrolidone / N-acryloxysuccinimide, le poly-6-aminoglucose, et les enzymes comme la peroxydase de raifort (HRP) ou la phosphatase alcaline ou leurs dérivés porteurs d'au moins une fonction réactive.

Par fonction réactive de la molécule organique, on entend soit une fonction réactive notamment choisie parmi les fonctions ester, halogénocarbonyle, sulfhydrile, disulfure, époxyde, halogénoalkyle et aldéhyde ; soit une fonction activable par un agent activant comme les carbodiimides ou des composés homo- ou hétéro-bifonctionnels. A titre d'exemple, une fonction activable est notamment choisie parmi les fonctions acide, amine et hydroxyle.

Les sites de liaison par covalence définis précédemment peuvent exister naturellement dans le matériel protéique. Ou bien ils peuvent être "rapportés" préalablement dans le matériel protéique, sous forme de tag, selon des techniques bien connues de l'homme du métier telles que celle employée pour la purification des protéines par le procédé IMAC (Immobilized Metal ion-Affinity Chromatography) sur des résines **(1,2).** A titre d'exemple, de tels sites peuvent être incorporés dans un matériel protéique et notamment une protéine, par génie génétique pour obtenir des protéines recombinantes.

Un tag peut être défini comme une séquence d'acides aminés rapportée, c'est-à-dire ajoutée à la structure originale du matériel protéique, qui est introduite en un lieu privilégié de ladite structure originale pour lui permettre d'être exposée de façon pertinente, en particulier vis-à-vis de sa fixation covalente sur la molécule organique.

Conformément à l'invention, un site de liaison par covalence du matériel protéique considéré peut consister en un tag comprenant six résidus lysine, ou dérivé de lysine, ou plus, et éventuellement, d'autres acides aminés, ou peut consister en plusieurs desdits tags.

Par dérivé de lysine, on comprend que la lysine peut être chimiquement modifiée, pour autant que ces modifications préservent substantiellement voire développent la spécificité du site de liaison par covalence. A titre d'exemple, on peut citer le remplacement de la L-lysine par la D-lysine, et vice-versa ; une modification de la chaîne latérale de la lysine, telle qu'une acétylation de la fonction amine, une estérification de la fonction carboxylique ; une modification des liaisons peptidiques telles que par exemple des liaisons carba, rétro, inverso, retro-inverso, réduites et méthylène-oxy.

Le ou les tags décrits ci-dessus peuvent se trouver à un endroit quelconque de la structure primaire du matériel protéique. De préférence, il est situé sur l'extrémité N- ou C-terminale du matériel protéique.

Selon la présente invention, on définit en outre différents procédés de mise en évidence d'un matériel biologique cible contenu dans un échantillon, en fonction notamment de sa nature :
- si le matériel biologique cible est un anticorps, le matériel protéique comprend un antigène qui reconnaît spécifiquement ledit antigène ;
- si le matériel biologique cible est un antigène, le matériel protéique comprend un anticorps qui reconnaît spécifiquement ledit antigène.

D'autres objets de l'invention sont ci-après exposés.

Ainsi l'invention concerne une phase de capture d'un matériel biologique cible qui comprend une molécule organique ayant au moins une fonction réactive et au moins un matériel protéique susceptible de reconnaître ou de se lier spécifiquement et directement ou indirectement, au matériel biologique cible, ledit matériel protéique possédant un site spécifique de liaison par covalence à la fonction réactive de la molécule organique, qui consiste en au moins un tag comprenant au moins six résidus lysine contigus ; préférentiellement, la molécule organique est un polymère ou un haptène.

Un polymère avantageux est particulaire ou linéaire et notamment choisi parmi les homopolymères tels que la polylysine, la polytyrosine ; et les copolymères tels que les copolymères d'anhydride maléique, les copolymères de N-vinyl-pyrrolidone, les polysaccharides, naturels ou synthétiques, les polynucléotides et les copolymères d'acides aminés comme les enzymes. Pus avantageusement encore, le polymère est choisi parmi le copolymère anhydride maléique / méthyl-vinyl-éther, le copolymère N-vinyl-pyrrolidone / N-acryloxysuccinimide, le poly-6-aminoglucose, la peroxydase de raifort (HRP) et la phosphatase alcaline.

Si la molécule organique est un haptène, la phase de capture peut en outre comprendre une molécule porteuse appropriée. Préférentiellement, l'haptène est la biotine, et le cas échéant la molécule porteuse est l'avidine.

Avantageusement, le tag tel que défini précédemment est placé sur l'extrémité N- ou C-terminale du matériel protéique.

La fonction réactive de la molécule organique est notamment choisie parmi les fonctions ester, acide, halogénocarbonyle, sulfhydrile, disulfure, époxyde, halogénoalkyle et aldéhyde.

L'invention concerne aussi une phase de détection d'un matériel biologique cible, présentant les mêmes caractéristiques que la phase de capture ci-dessus et comprenant en outre un marqueur.

Comme indiqué précédemment, la molécule organique peut être un polymère ou un haptène. Selon la seconde possibilité, la molécule organique consistant en l'haptène représente aussi le marqueur.

Le marqueur de la phase de détection est de préférence choisi dans le groupe consistant en une enzyme, une protéine, un peptide, un anticorps, un haptène tel que la biotine, l'iminobiotine, un composé fluorescent tel que la rhodamine, un composé radioactif, un composé chémioluminescent, un composant d'électrodensité, un composant magnétique et analogues.

Un autre objet de l'invention est un réactif pour la mise en évidence d'un matériel biologique cible, comprenant une phase de capture de l'invention, et/ou une phase de détection de l'invention.

Avantageusement la phase de capture est fixée, directement ou indirectement, sur un support solide, par adsorption passive ou par covalence.

Le support solide peut se présenter sous toutes formes appropriées telles qu'une plaque, un cône, une bille éventuellement radioactive et/ou fluorescente et/ou magnétique et/ou conductrice, une barrette, un tube de verre, un puits, une feuille, une puce ou analogue. Il est choisi parmi les polystyrènes, les copolymères styrène-butadiène, les copolymères styrène-butadiène en mélange avec des polystyrènes, des polypropylènes, des polycarbonates, des copolymères polystyrène-acrylonitrile, des copolymères styrène-méthylméthacrylate de méthyle, parmi les fibres synthétiques et naturelles, parmi les polysaccharides et les dérivés de la cellulose, le verre, le silicium et leurs dérivés.

Comme ce sera mis en évidence dans la partie expérimentale qui suit, les auteurs ont obtenu des anticorps monoclonaux dirigés contre les tags de tels que définis précédemment et en particulier contre le tag qui consiste en une suite de six résidus lysine.

On peut ainsi envisager l'utilisation d'un tel tag dans un procédé de mise en évidence d'un matériel biologique selon lequel :
on dispose d'une phase de capture, on met en contact ledit matériel biologique cible avec au moins la phase de capture, et on détecte le matériel biologique cible fixé sur la phase de capture, procédé selon lequel la phase de capture comprend une molécule organique qui comprend ou consiste en un anticorps anti-tag et au moins un matériel protéique susceptible de reconnaître ou de se lier, spécifiquement et directement ou indirectement, au matériel biologique cible, ledit matériel protéique possédant un site spécifique de liaison à la molécule organique, qui consiste en au moins un tag comprenant au moins six résidus lysine, ou dérivé de lysine, contigus.

Un tel procédé peut en outre comprendre l'utilisation d'une phase de détection, présentant les caractéristiques de la phase de capture telle qu'elle vient d'être définie, et comprenant en outre un marqueur. Ce marqueur peut avantageusement consister en undit anticorps.

Les caractéristiques et avantages des différents objets de l'invention sont ci-après illustrés, à l'appui des Exemples 1 à 6 et des Figures 1 à 7.

La Figure 1 représente un graphe traduisant le rendement de couplage par liaison covalente entre la protéine RH24K (en clair) et la protéine RH24 (en foncé) et le copolymère, pour différentes conditions de couplage ; Acet = acétate ; Phos = phosphate ; Bor = borate ; Carb = carbonate.

La Figure 2 représente un graphe de comparaison entre la stabilité en fonction du temps (en jours), du composé conjugué polymère (AMVE) / protéine (RH24K) (courbe ■) et celle de la protéine RH24K libre (courbe ●).

La Figure 3 représente un schéma résumant la technique de détection "sandwich".

La Figure 4 représente un graphe traduisant l'immunoréactivité des composés conjugués polymère / RH24 (courbe ●), et polymère / RH24K (courbe ■), vis-à-vis d'un anticorps monoclonal.

La Figure 5 représente un graphe traduisant l'immunoréactivité des composés conjugués polymère / RH24 (courbe ●), et polymère / RH24K (courbe ■), vis-à-vis d'un sérum.

La Figure 6 illustre les résultats d'un test ELISA sandwich et indirecte avec l'anticorps IG2D4, à l'aide d'un graphe représentant la DO en fonction du titre de l'anticorps.

La Figure 7 illustre les résultats d'un test ELISA indirecte avec l'anticorps IG2D4, à l'aide d'un graphe représentant la DO en fonction du titre de l'anticorps.

### Exemple 1 : Expression et purification de la RH24K:

Le pMH24 (Cheynet et al. 1993) a été modifié par insertion d'un adaptateur synthétique en 3' du gène de la p24 en vue d'exprimer la RH24K, correspondant à la RH24 qui présente sur son extrémité C-terminale un site spécifique de liaison par covalence dénommé "queue polylysine" et constitué par 6 résidus lysine contigus.

L'adaptateur a préalablement été réalisé par hybridation de deux oligonucléotides ayant respectivement les séquences suivantes : 
SEQ ID NO: 1 :
5' GGGAAGAAGA AGAAGAAGAA GTCTGTCGAC GAATCTCT 3'
 
SEQ ID NO: 2 :
5' CTAGAGAGAT TCGTCGACAG ACTTCTrCTT CTTCTTC'UCC C 3'
 en tampon Tris 10 mM NaCl 50 mM, DTE 1 mM, MgCl₂ 10 mM pH 7,5, par chauffage à 70°C pendant 5 minutes puis refroidissement lent d'abord à 37°C pendant 30 minutes puis à température ambiante pendant 30 minutes. Cet adaptateur a ensuite été cloné entre les sites SmaI et XbaI du pMH24 en 3' du gène de la RH24. Le plasmide résultant, le pMH24K3', a été amplifié dans la souche bactérienne *E. coli* XLI (recA1, endA1, gyrA96, thi-1, hsdRI7, supE44, relA1, lac [F' proAB, lacIPZΔM15, Tn10(tet^{r})] puis séquencé par la méthode Taq Dye Deoxy Terminator Matrix Standart (Applied Biosystem). La séquence obtenue ainsi que la séquence correspondante sur la protéine obtenue en C-terminal sont données ci-dessous:

SmaI
5'ATG CCC GGG AAG AAG AAG AAG AAG AAG
Nter Met... [247 acides aminés]...Pro Gly Lys Lys Lys Lys Lys Lys
XbaI
TCT GTC GAC GAA TCT CTC TAG A 3'
Ser Val Asp Glu Ser Leu Cter

La protéine est exprimée sous le contrôle du promoteur Tac dans la souche bactérienne *E. coli* XLI (voir ci-dessus). Une préculture de *E. coli* XLI, contenant le vecteur d'expression, est préalablement effectuée pendant une nuit à 37°C dans du Luria broth (LB) en présence d'ampicilline (amp; 50 µg/ml) et de tétracycline (tet; 12 µg/ ml) afin d'ensemencer du milieu LB-amp-tet (1/40 au 1/25); ce dernier est mis en culture jusqu'à obtenir une DO à 600 nm de 0,6. L'expression de la protéine est alors induite par addition dans le milieu de isopropyl-β-D-thiogalactopyranoside (IPTG) à une concentration de 1 mM pendant 2h30 à 37°C sous agitation. Après centrifugation à 5000 tours/min (rotor JA21, Beckman) pendant 10 minutes à 4°C, les bactéries sont reprises dans un tampon Tris-HCl 50 mM EDTA, 1 mM NaCl 100 mM MgCl₂ 10 mM pH 8 (3 ml/g de biomasse) en présence d'inhibiteur de protéase (aprotinine 2 µg/ml et leupeptine 2 µg/ml) puis lysées par sonication à 0°C. La fraction soluble est reprise par centrifugation à 10 000 tours/min (rotor JA21, Beckman) pendant 15 minutes à 4°C puis déposée sur une colonne d'affinité par chélation métallique en vue de la purification de la RH24K (Chelating Sepharose Fast Flow no. 17057501). Le gel a préalablement été chargé en Zn²⁺ suivant les recommandations du fournisseur puis équilibré en tampon phosphate de sodium 67 mM NaCl pH 7,8 en vue de la purification. Après dépôt du surnageant, la colonne est successivement lavée par un tampon phosphate de sodium 67 mM NaCl pH 7,8 puis acétate d'ammonium 200 mM NaCl 0,5 M pH 6. La RH24K est éluée par l'acétate d'ammonium 200 mM NaCl 0,5 M pH 4. Les fractions recueillies sont dialysées contre du tampon phosphate de sodium 50 mM pH 7,8.

La protéine purifiée est caractérisée par électrophorèse sur SDS-PAGE et par passage en gel filtration / HPLC. Sa pureté est supérieure à 90 % donc comparable à la pureté de la RH24. Analysée en spectrométrie de masse, son poids moléculaire est de 28027 compatible avec le poids moléculaire théorique 27992. La protéine est reconnue par des anticorps anti-p24 polyclonaux et monoclonaux en Western Blot et en ELISA.

### Exemple 2 : Couplage de la RH24K et de la RH24 sur le copolymère AMVE

A une solution de protéine dans un tampon approprié, à la concentration de 1 g/l, on ajoute 5 µl de solution de polymère à la concentration de 1 g/l.

Le milieu réactionnel est agité 3 heures à 37°C.

L'avancement de la réaction est suivi en HPLC, sur une colonne d'exclusion stérique Waters avec comme phase mobile un tampon phosphate 0,1 M pH 6,8.

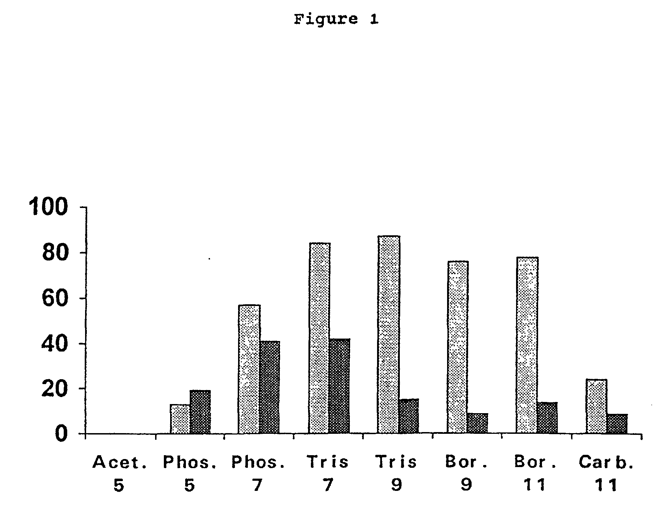

Les rendements de couplages rapportés sur la figure 1 montrent que la protéine modifiée se couple préférentiellement au polymère et dans un plus large spectre de conditions expérimentales.

### Exemple 3 : Effet de la nature du polymère sur le rendement de couplage

L'effet de la nature du comonomère entrant dans la composition du polymère est évalué dans les mêmes conditions que celles décrites ci dessus.

Les résultats sont rapportés dans le tableau ci-dessous.

**TABLEAU**

| Polymère | Rendement de couplage (%) |
|---|---|
| AMVE poly(méthylvinyléther/anhydride maléique) | 65 |
| PEAM poly(éthylène/anhydride maléfique) | **86** |
| SAM poly(styrène/anhydride maléique) | 49 |
| NVPAM poly(N-vinylpyrrolidone/anhydride maléique) | 36 |

L'effet du comonomère est assez prononcé puisque les rendements de couplage varient dans un domaine compris entre 36 et 86 % selon la nature du comonomère.

### Exemple 4 : Stabilité comparée des composés conjugués protéines/polymères vis-à-vis de la protéine seule

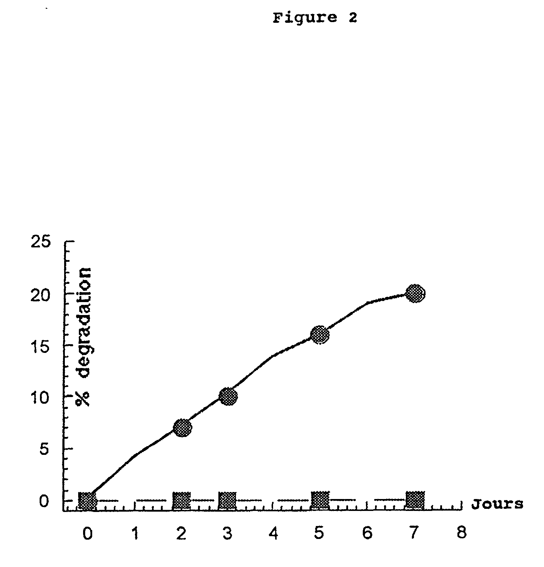

Les composés conjugués ont été stockés à 37°C dans le milieu de couplage et analysés par chromatographie d'exclusion stérique afin d'évaluer la quantité de protéine résiduelle dans le milieu de couplage. Les résultats sont rapportés dans le graphe représenté sur la Figure 2.

Le couplage sur polymère confère ainsi à la protéine une meilleurs stabilité au stockage.

### Exemple 5 : Biotinylation orientée de la RH24K

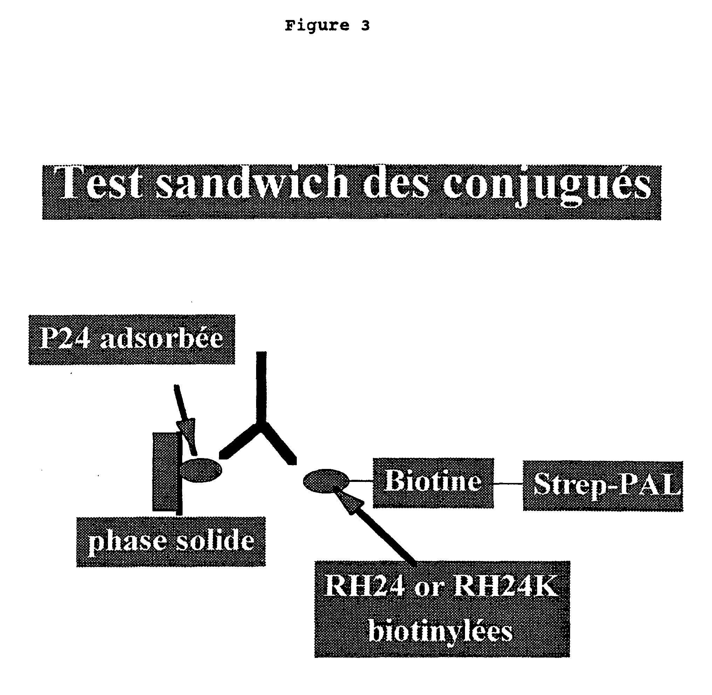

Les composés conjugués réalisés sont des p24 (RH24 ou RH24K) biotinylées qui seront utilisés en détection dans le test sandwich antigène p24, conformément au schéma représenté sur la Figure 3.

Les couplages de la biotine sont effectués sur la RH24K et sur la RH24, contrôlés, puis analysés en test sandwich antigène de manière à définir des conditions de couplage favorables à la RH24K.

Les protéines sont utilisées à une concentration de 1 mg/ml. Différents tampons ont été testés sur la base des tampons testés lors des couplages p24 / AMVE : borate 0,1 M pH 9,2, Tris 0,1 M pH 7,2, phosphate 0,1 M pH 7,2 et carbonate 0,1 M pH 8,5. Les rapports biotine / p24 suivants ont été testés: 5/1, 10/1, 25/1, 50/1, 100/1. Les couplages ont été incubés pendant 1 heure à 37°C.

Les composés conjugués p24 / biotine ont été utilisés en détection suivant le schéma représenté sur la Figure 3 avec des anticorps monoclonaux et un sérum positif.

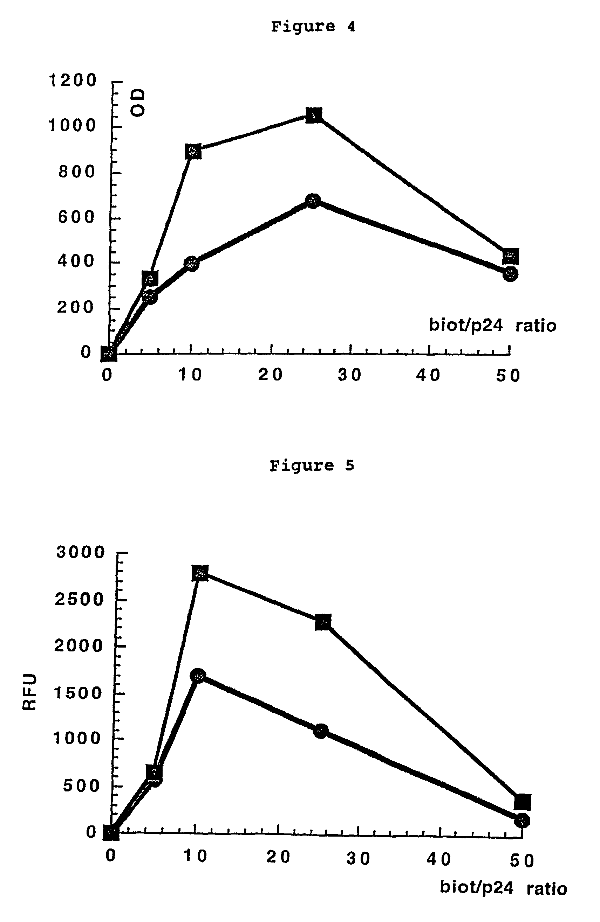

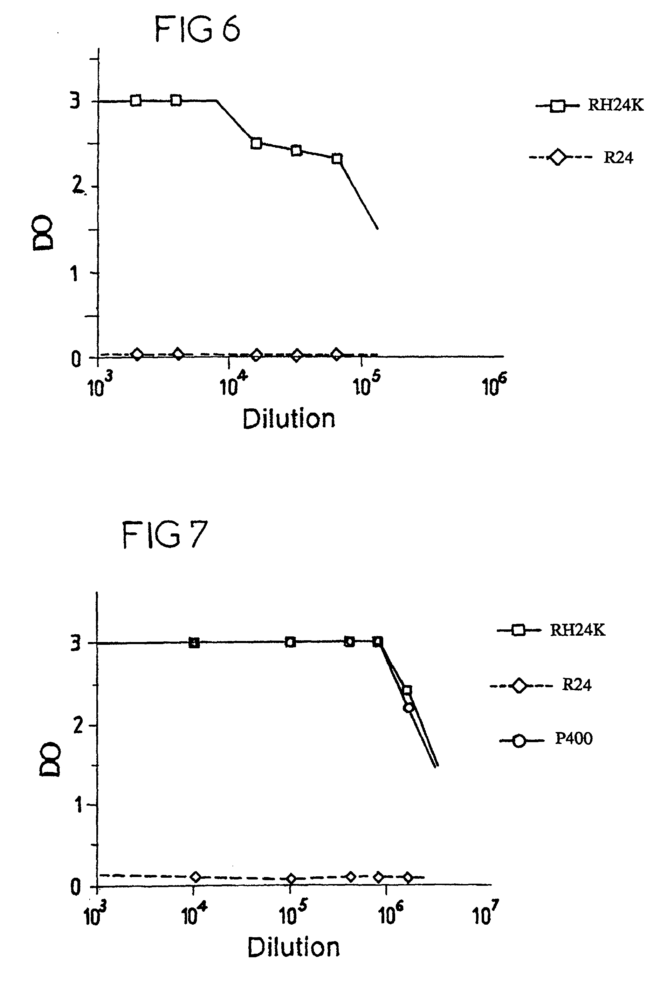

Les composés conjugués RH24 / biotine et RH24K / biotine montrent une activité en test sandwich antigène avec les anticorps monoclonaux et le sérum positif. A titre d'illustration, les Figures 4 et 5 montrent sous la forme de graphe, les réponses comparées des composés conjugués obtenus avec la RH24K et la RH24 à différents taux de fonctionnalisation en biotine. La Figure 4 concerne les résultats obtenus avec un anticorps monoclonal, test pratiqué en plaque de micro-titration. La Figure 5 représente les résultats obtenus sur sérum avec l'automate d'immunoanylyse Vidas de bioMérieux.

La RH24K présente un signal supérieur à celui de la RH24 quelles que soient les conditions expérimentales testées. On observe un rapport biotine / p24 optimal de 25 / 1 pour les anticorps monoclonaux, et de 10 / 1 pour le sérum.

### Exemple 6 : Influence de la composition en résidus lysine du tag poly-lysine sur l'efficacité de couplage

Le matériel biologique utilisé pour évaluer l'influence de la composition en résidus lysine du tag poly-lysine sur l'efficacité de couplage est le suivant :
- la protéine recombinante RH24K ; elle comporte, dans sa partie amino-terminale, un tag poly-histidine [MRGS(H)₆GSVDESM] servant à sa purification par chélation avec un ion métallique, et, dans sa partie carboxy terminale, un tag poly-lysine [PG(K)₆SVDESL] dédié au couplage ; cette protéine peut être représentée comme suit :
   MRGS(H)₆GSVDESM-p24-PG(K)₆SVDESL)
- la protéine recombinante RHK24 ; elle comporte, dans sa partie amino-terminale, un tag poly-lysine [MRGSCH(K)₂HH(K)₂HH(K)₂GSVDESM] ; cette protéine peut être représentée comme suit :
   MRGSCH(K)₂HH(K)₂HH(K)₂GSVDESM-p24
- la protéine recombinante R24 ; elle ne contient ni le tag poly-lysine carboxy-terminal, ni le tag poly-histidine amino-terminal ; on peut la représenter par p24.

Les polymères utilisés sont l'AMVE (anhydride maléique-co-méthylvinyléther). Les conditions de couplage sont identiques pour les trois protéines.

Deux tampons de couplage ont été utilisés, le tampon carbonate 0,1 M pH 8,2 et le Tris HCl 0,05 M pH 9,0.

Les résultats sont présentés dans le tableau suivant illustrant le rendement de couplage des protéines recombinantes sur le polymère AMVE effectué dans deux conditions réactionnelles différentes. Ils montrent l'importance de la contiguïté des résidus lysine.

En effet, le rendement de couplage obtenu avec la protéine RH24K (6 résidus lysine contigus) est très supérieur au rendement de couplage obtenu avec la protéine RHK24 (6 résidus lysine en trois blocs de 2).

En fait, le rendement de couplage obtenu avec la protéine RHK24 (6 résidus lysine en trois blocs de 2) est similaire à celui obtenu avec la protéine R24 (pas de tag).

**TABLEAU**

| Protéine TAG poly-lysine | R24 pas de TAG | RH24K PG(K)₆SVDESL | RHK24 MRGSCH(K)₂HH(K)₂HH(K)₂GSVDESM] |
|---|---|---|---|
| Tampon carbonate 0,1 M pH 8,2 | 30 % | 95 % | 33 % |
| Tampon Tris HCl 0,05M pH 9,0 | 35 % | 100 % | 20 % |

### Exemple 7 : Obtention d'anticorps anti-tag poly-lysine (K)₆SVDESL

Le matériel biologique utilisé pour obtenir des anticorps anti-tag pouvant reconnaître ce tag dans le contexte d'une fusion avec une protéine recombinante est le suivant :
- la protéine recombinante RH24K ; celle-ci comporte, dans sa partie amino-terminale, un tag poly-histidine [MRGS(H)₆GSVDESM] servant à sa purification par chélation avec un ion métallique et dans sa partie carboxy terminale un tag poly-lysine [PG(K)₆SVDESL] dédié au couplage et représentée par
   MRGS(H)₆GSVDESM-p24-PG(K)₆SVDESL
- le peptide P400, dont la séquence est C(K)₆SVDESL, couplé à la KLH (P400-KLH).

Le matériel biologique utilisé pour sélectionner les anticorps produit est le suivant:
- la protéine recombinante RH24K
- la protéine recombinante RH24 ne contenant pas de tag poly-lysine carboxy-terminal, et représentée par
   MRGS(H)₆GSVDESM-p24)
- la protéine recombinante RH24 ; elle ne contient ni le tag poly-lysine carboxy-terminal, ni le tag poly-histidine amino-terminal, et représentée par p24.
- le peptide P400, dont la séquence est C(K)₆SVDESL.

Les deux formats de test suivants ont été utilisés pour sélectionner les anticorps monoclonaux :
- un test ELISA sandwich, comprenant en phase de capture, un anticorps de chèvre anti-souris et, en phase de détection, les antigènes RH24K ou R24 révélés par un anticorps monoclonal anti-24 couplé à la peroxydase ;
- un test ELISA indirect, comprenant en phase de capture, les antigènes RH24K ou R24 ou P400 et, en phase de détection, un anticorps de chèvre anti-IgV de souris couplé à la peroxydase.

Des souris BALB/c et A/J ont été immunisées selon le protocole suivant : 3 injections intra-péritonéales, avec, pour première injection, la protéine RH24K, et pour les deux injections suivantes, le peptide 400 couplé à la KLH. Des fusions ont été réalisées et les criblages réalisés à l'aide des tests ELISA sandwich et indirect décrits ci-dessus.

80 hybrides ont été sélectionnés suivant les critères suivants :
- reconnaissance du peptide P400 (C(K)₆SVDESL)
- reconnaissance de la protéine RH24K (MRGS(H)₆GSVDESM-p24-PG(K)₆SVDESL)
- non reconnaissance de la protéine RH24 (MRGS(H)₆GSVDESM-p24)
- non reconnaissance de la protéine R24.

Ces anticorps reconnaissent donc la séquence (K)₆SVDESL fusionnée à une protéine mais non la séquence MRGS(H)₆GSVDESM comportant également le motif SVDESL ni la protéine p24.

Cinq d'entre eux ont été clonés et produits sous forme d'ascites: il s'agit des anticorps IG2D4, 2G2B3, 2G4A12, 5F12ES, 14E1G7, tous d'isotype IgG1 k.

Les résultats des tests ELISA sandwich et indirect de l'anticorps IG2D4 sont illustrés dans les figures 6 et 7. La figure 6 illustre un test ELISA sandwich et représente la DO en fonction du titre (dilution) de l'ascite de l'anticorps IG2D4 ; les protéines recombinantes RH24K et R24, constituant la phase de détection, sont utilisées à la concentration de 0,1 µg/ml. La figure 7 illustre un test ELISA indirect et représente la DO en fonction du titre (dilution) de l'ascite de l'anticorps IG2D4 ; les protéines recombinantes RH24K et R24, constituant la phase de capture, sont utilisées à la concentration de 0,5 µg/ml et le peptide de synthèse P400 à la concentration de 0,05 µg/ml.

### BIBLIOGRAPHIE

(1) Porath J., Carlsson., Olsson., Belfrage J., *Nature,* **258,** 598 (1975)
(2) Porath J., *Trends Anal. Chem.,* **7,** 254 (1988)
(3) Cheynet, *Protein Expression and Purification,* **4,** 367-372 (1993)

## Revendications

1. Procédé de mise en évidence d'un matériel biologique cible contenu dans un échantillon, selon lequel on dispose d'une phase de capture, on met en contact ledit matériel biologique cible avec au moins la phase de capture, et on détecte le matériel biologique cible fixé sur la phase de capture,
**ledit procédé étant caractérisé en ce que**,
la phase de capture comprend une molécule organique ayant au moins une fonction réactive et au moins un matériel protéique susceptible de reconnaître ou de se lier, spécifiquement et directement ou indirectement, au matériel biologique cible, ledit matériel protéique possédant un site spécifique de liaison par covalence à la fonction réactive de la molécule organique, qui consiste en au moins un tag comprenant au moins six résidus lysine, ou dérivé de lysine, contigus.

2. Procédé selon la revendication 1, **caractérisé en ce que** la phase de capture comprend en outre un marqueur.

3. Procédé selon la revendication 2, **caractérisé en ce que** la phase de capture est une phase de détection.

4. Procédé selon la revendication 1, **caractérisé en ce qu'**on dispose en outre d'une phase de détection qui comprend une molécule organique ayant au moins une fonction réactive, au moins un matériel protéique susceptible de reconnaître ou de se lier, spécifiquement et directement ou indirectement, au matériel biologique cible, et un marqueur, ledit matériel protéique possédant un site spécifique de liaison par covalence à la fonction réactive de la molécule organique, qui consiste en au moins un tag comprenant au moins six résidus lysine, ou dérivé de lysine, contigus.

5. Procédé selon la revendication 4, **caractérisé en ce que** la molécule organique et le matériel protéique de la phase de détection sont respectivement identiques à et/ou différents de la molécule organique et du matériel protéique de la phase de capture.

6. Procédé selon les revendications 1 et/ou 4, **caractérisé en ce que** la molécule organique est un polymère, particulaire ou linéaire.

7. Procédé selon la revendication 6, **caractérisé en ce que** le polymère est choisi parmi les homopolymères tels que la polylysine, la polytyrosine ; et les copolymères tels que les copolymères d'anhydride maléique, les copolymères de N-vinyl-pyrrolidone, les polysaccharides, naturels ou synthétiques et les polynucléotides et les copolymères d'acides aminés comme les enzymes.

8. Procédé selon la revendication 7, **caractérisé en ce que** le polymère est choisi parmi le copolymère anhydride maléique / méthyl-vinyl-éther, le copolymère N-vinyl-pyrrolidone / N-acryloxysuccinimide, le poly-6-aminoglucose.

9. Procédé selon les revendications 1 et/ou 4, **caractérisé en ce que** la molécule organique est un haptène.

10. Procédé selon la revendication 9, **caractérisé** en qu'on fixe la molécule organique sur une molécule porteuse.

11. Procédé selon la revendication 9, **caractérisé en ce que** la molécule organique est la biotine et la molécule porteuse est l'avidine.

12. Procédé selon la revendication 4, **caractérisé en ce que** la molécule organique de la phase de détection est le marqueur.

13. Procédé selon les revendications 1 et/ou 4, **caractérisé en ce que** le tag est placé sur l'extrémité N-ou C-terminale du matériel protéique.

14. Procédé selon les revendications 1 et/ou 4, **caractérisé en ce que** la fonction réactive de la molécule organique est choisie parmi les fonctions ester, acide, halogénocarbonyle, sulfhydrile, disulfure, époxyde, halogénoalkyle et aldéhyde.

15. Procédé selon l'une quelconque des revendications 1 à 14, **caractérisé en ce que** le matériel protéique comprend un antigène et le matériel biologique est un anticorps spécifiquement reconnu par ledit antigène.

16. Procédé selon l'une quelconque des revendications 1 à 14, **caractérisé en ce que** le matériel protéique comprend un anticorps et le matériel biologique est un antigène spécifiquement reconnu par ledit anticorps.

17. Phase de capture d'un matériel biologique cible **caractérisée en ce qu'**elle comprend une molécule organique ayant au moins une fonction réactive et au moins un matériel protéique susceptible de reconnaître ou de se lier spécifiquement et directement ou indirectement, au matériel biologique cible, ledit matériel protéique possédant un site spécifique de liaison par covalence à la fonction réactive de la molécule organique, qui consiste en au moins un tag comprenant au moins six résidus lysine, ou dérivé de lysine, contigus.

18. Phase de détection d'un matériel biologique cible, **caractérisée en ce qu'**elle comprend une molécule organique ayant au moins une fonction réactive, au moins un matériel protéique susceptible de reconnaître ou de se lier, spécifiquement et directement ou indirectement, au matériel biologique cible, et un marqueur, ledit matériel protéique possédant un site spécifique de liaison par covalence à la fonction réactive de la molécule organique, qui consiste en au moins un tag comprenant au moins six résidus lysine, ou dérivé de lysine, contigus.

19. Phase de capture ou de détection selon la revendication 17 ou 18, **caractérisée en ce que** la molécule organique est un polymère, particulaire ou linéaire.

20. Phase de capture ou de détection selon la revendication 19, **caractérisée en ce que** le polymère est choisi parmi les homopolymères tels que la polylysine, la polytyrosine ; et les copolymères tels que les copolymères d'anhydride maléique, les copolymères de N-vinyl-pyrrolidone, les polysaccharides, naturels ou synthétiques, les polynucléotides et les copolymères d'acides aminés comme les enzymes.

21. Phase de capture ou de détection selon la revendication 20, **caractérisée en ce que** le polymère est choisi parmi le copolymère anhydride maléique / méthyl-vinyl-éther, le copolymère N-vinyl-pyrrolidone / N-acryloxysuccinimide, le poly-6-aminoglucose, la peroxydase de raifort (HRP) et la phosphatase alcaline.

22. Phase de capture selon la revendication 21, **caractérisée en ce que** la molécule organique est un haptène, tel que la biotine.

23. Phase de capture ou de détection selon la revendication 17 ou 18, **caractérisée en ce que** le tag est placé sur l'extrémité N- ou C-terminale du matériel protéique.

24. Phase de capture ou de détection selon la revendication 17 ou 18, **caractérisée en ce que** la fonction réactive de la molécule organique est choisie parmi les fonctions ester, acide, halogénocarbonyle, sulfhydrile, disulfure, époxyde, halogénoalkyle et aldéhyde.

25. Phase de détection selon la revendication 18, **caractérisée en ce que** la molécule organique est le marqueur.

26. Phase de détection selon la revendication 18 ou 25, **caractérisée en ce que** le marqueur est choisi dans le groupe consistant en une enzyme, une protéine, un peptide, un anticorps, un haptène tel que la biotine, l'iminobiotine, un composé fluorescent tel que la rhodamine, un composé radioactif, un composé chémioluminescent, un composant d'électrodensité, un composant magnétique et analogues.

27. Réactif pour la mise en évidence d'un matériel biologique cible, comprenant une phase de capture selon la revendication 17 et l'une quelconque des revendications 19 à 24, et/ou une phase de détection selon l'une quelconque des revendications 18 à 26.

28. Réactif selon la revendication 27, **caractérisé en ce que** la phase de capture est fixée, directement ou indirectement, sur un support solide, par adsorption passive ou par covalence.

## Patentansprüche

1. Verfahren zum Aufzeigen eines in einer Probe enthaltenen biologischen Zielmaterials, bei dem eine Einfangphase bereitgestellt wird, das biologische Zielmaterial zumindest mit der Einfangphase in Kontakt gebracht wird, und das an der Einfangphase fixierte biologische Zielmaterial detektiert wird,
**wobei das Verfahren dadurch gekennzeichnet ist, dass**
die Einfangphase ein organisches Molekül mit zumindest einer reaktiven Gruppe und zumindest ein Proteinmaterial aufweist, das in der Lage ist, das biologische Zielmaterial zu erkennen oder spezifisch und direkt oder indirekt daran zu binden, wobei das Proteinmaterial eine spezifische Stelle für kovalente Bindung mit der reaktiven Gruppe des organischen Moleküls besitzt, die aus wenigstens einem Schwanz besteht, der wenigstens sechs zusammenhängende Lysin-Reste oder Lysinderivate umfasst.

2. Verfahren nach Anspruch 1, **dadurch gekennzeichnet, dass** die Einfangphase unter anderem einen Marker enthält.

3. Verfahren nach Anspruch 2, **dadurch gekennzeichnet, dass** die Einfangphase eine Detektionsphase ist.

4. Verfahren nach Anspruch 1, **dadurch gekennzeichnet, dass** unter anderem eine Detektionsphase bereitgestellt wird, die ein organisches Molekül mit wenigstens einer reaktiven Gruppe, zumindest ein Proteinmaterial, das in der Lage ist, das biologische Zielmaterial zu erkennen oder sich spezifisch und direkt oder indirekt daran zu binden, sowie einen Marker enthält, wobei das Proteinmaterial eine spezifische Stelle für kovalente Bindung an die reaktive Gruppe des organischen Moleküls besitzt, die aus wenigstens einem Schwanz besteht, der zumindest sechs zusammenhängende Lysin-Reste oder Lysinderivate umfasst.

5. Verfahren nach Anspruch 4, **dadurch gekennzeichnet, dass** das organische Molekül und das Proteinmaterial der Detektionsphase jeweils identisch zu oder verschieden von dem organischen Material und dem Proteinmaterial der Einfangphase sind.

6. Verfahren nach den Ansprüchen 1 und/oder 4, **dadurch gekennzeichnet, dass** das organische Molekül ein partikelartiges oder lineares Polymer ist.

7. Verfahren nach Anspruch 6, **dadurch gekennzeichnet, dass** das Polymer ausgewählt ist aus den Homopolymeren, wie Polylysin und Polytyrosin, sowie den Copolymeren, wie den Copolymeren von Maleinsäureanhydrid, den Copolymeren von N-vinyl-pyrrolidon, den natürlichen oder synthetischen Polysacchariden, den Polynukleotiden und den Copolymeren der Aminosäuren, wie den Enzymen.

8. Verfahren nach Anspruch 7, **dadurch gekennzeichnet, dass** das Polymer ausgewählt ist aus dem Copolymer Maleinsäureanhydrid/Methyl-vinyl-ether, dem Copolymer N-vinyl-pyrrolidon/N-acryloxysuccinimid, der Poly-6-aminoglucose.

9. Verfahren nach den Ansprüchen 1 und/oder 4, **dadurch gekennzeichnet, dass** das organische Molekül ein Hapten ist.

10. Verfahren nach Anspruch 9, **dadurch gekennzeichnet, dass** das organische Molekül auf einem Trägermolekül fixiert wird.

11. Verfahren nach Anspruch 9, **dadurch gekennzeichnet, dass** das organische Molekül Biotin und das Trägermolekül Avidin ist.

12. Verfahren nach Anspruch 4, **dadurch gekennzeichnet, dass** das organische Molekül der Detektionsphase der Marker ist.

13. Verfahren nach den Ansprüchen 1 und/oder 4, **dadurch gekennzeichnet, dass** der Schwanz an dem N- oder C-terminalen Ende des Proteinmaterials angeordnet ist.

14. Verfahren nach einem der Ansprüche 1 und/oder 4, **dadurch gekennzeichnet, dass** die reaktive Gruppe des organischen Moleküls ausgewählt ist aus den Gruppen Ester, Säure, Halogencarbonyl, Sulfhydryl, Sulfid, Epoxid, Halogenalkyl und Aldehyd.

15. Verfahren nach einem der Ansprüche 1 bis 14, **dadurch gekennzeichnet, dass** das Proteinmaterial ein Antigen umfasst und dass das biologische Material ein Antikörper ist, der spezifisch durch das genannte Antigen erkannt wird.

16. Verfahren nach einem der Ansprüche 1 bis 14, **dadurch gekennzeichnet, dass** das Proteinmaterial einen Antikörper umfasst und dass das biologische Material ein Antigen ist, das spezifisch durch den genannten Antikörper erkannt wird.

17. Einfangphase für ein biologisches zielmaterial, **dadurch gekennzeichnet, dass** sie ein organisches Molekül mit zumindest einer reaktiven Gruppe und zumindest ein Proteinmaterial aufweist, das dazu in der Lage ist, das biologische Zielmaterial zu erkennen oder spezifisch und direkt oder indirekt daran zu binden, wobei das Proteinmaterial eine spezifische Stelle für kovalente Bindung an die reaktive Gruppe des organischen Moleküls umfasst, die aus wenigstens einem Schwanz besteht, der wenigstens sechs zusammenhängende Lysin-Reste oder Lysinderivate aufweist.

18. Detektionsphase für ein biologisches Zielmaterial, **dadurch gekennzeichnet, dass** sie ein organisches Molekül mit einer reaktiven Gruppe, wenigstens ein Proteinmaterial, das dazu in der Lage ist, das biologische Zielmaterial zu erkennen oder spezifisch und direkt oder indirekt daran zu binden, sowie einen Marker aufweist, wobei das Proteinmaterial eine spezifische Stelle für kovalente Bindung an die reaktive Gruppe des organischen Moleküls besitzt, die aus wenigstens einem Schwanz besteht, der zumindest sechs zusammenhängende Lysin-Reste oder Lysinderivate umfasst.

19. Einfang- oder Detektionsphase nach Anspruch 17 oder 18, **dadurch gekennzeichnet, dass** das organische Molekül ein partikelartiges oder lineares Polymer ist.

20. Einfang- oder Detektionsphase nach Anspruch 19, **dadurch gekennzeichnet, dass** das Polymer ausgewählt ist aus den Homopolymeren, wie Polylysin und Polytyrosin, sowie den Copolymeren, wie den Copolymeren von Maleinsäureanhydrid, den Copolymeren von N-vinyl-pyrrolidon, den natürlichen oder synthetischen Polysacchariden, den Polynukleotiden und den Copolymeren der Aminosäuren, wie den Enzymen.

21. Einfang- oder Detektionsphase nach Anspruch 20, **dadurch gekennzeichnet, dass** das Polymer ausgewählt ist aus dem Copolymer Maleinsäureanhydrid/Methyl-vinyl-ether, dem Copolymer N-vinyl-pyrrolidon/N-acryloxysuccinimid, der Poly-6-aminoglucose, Meerrettichperoxidase (HRP) und alkalische Phosphatase.

22. Einfangphase nach Anspruch 21, **dadurch gekennzeichnet, dass** das organische Molekül ein Hapten ist, wie beispielsweise Biotin.

23. Einfang- oder Detektionsphase nach Anspruch 17 oder 18, **dadurch gekennzeichnet, dass** der Schwanz an dem N- oder C-terminalen Ende des Proteinmaterials angeordnet ist.

24. Einfang- oder Detektionsphase nach Anspruch 17 oder 18, **dadurch gekennzeichnet, dass** die reaktive Gruppe des organischen Moleküls ausgewählt ist aus den Gruppen Ester, Säure, Halogencarbonyl, Sulfhydryl, Sulfid, Epoxid, Halogenalkyl und Aldehyd.

25. Detektionsphase nach Anspruch 18, **dadurch gekennzeichnet, dass** das organische Molekül der Marker ist.

26. Detektionsphase nach Anspruch 18 Oder 25, **dadurch gekennzeichnet, dass** der Marker ausgewählt ist aus der Gruppe, bestehend aus einem Enzym, einem Protein, einem Peptid, einem Antikörper, einem Hapten wie beispielsweise Biotin, Iminobiotin, einer fluoreszierenden Zusammensetzung wie beispielsweise Rhodamin, einer radioaktiven Zusammensetzung, einer chemolumineszenten Zusammensetzung, einer Elektrodichte-Komponente, einer magnetischen Komponente und Analogen.

27. Reagens für das Aufzeigen eines biologischen Zielmaterials, mit einer Einfangphase nach Anspruch 17 und einem der Ansprüche 19 bis 24 und/oder einer Detektionsphase nach einem der Ansprüche 18 bis 26.

28. Reagens nach Anspruch 27, **dadurch gekennzeichnet, dass** die Einfangphase direkt oder indirekt an einem festen Träger durch passive Adsorption oder durch Kovalenz fixiert ist.

## Claims

1. Method for isolating a target biological material contained in a sample, according to which a capture phase is provided, said target biological material is placed in contact with at least the capture phase, and the target biological material bound to the capture phase is detected,
**said method being characterized in that**
the capture phase comprises an organic molecule containing at least one reactive function and at least one protein material capable of recognizing or binding, specifically and directly or indirectly, to the target biological material, said protein material containing a specific site for covalent binding to the reactive function of the organic molecule, which consists of at least one tag comprising at least six contiguous lysine or lysine-based residues.

2. Method according to Claim 1, **characterized in that** the capture phase also comprises a label.

3. Method according to Claim 2, **characterized in that** the capture phase is a detection phase.

4. Method according to Claim 1, **characterized in that** a detection phase is also provided, which comprises an organic molecule containing at least one reactive function, at least one protein material capable of recognizing or binding, specifically and directly or indirectly, to the target biological material, and a label, said protein material containing a specific site for covalent binding to the reactive function of the organic molecule, which consists of at least one tag comprising at least six contiguous lysine or lysine-based residues.

5. Method according to Claim 4, **characterized in that** the organic molecule and the protein material in the detection phase are, respectively, identical to and/or different from the organic molecule and the protein material in the capture phase.

6. Method according to Claims 1 and/or 4, **characterized in that** the organic molecule is a particulate or linear polymer.

7. Method according to Claim 6, **characterized in that** the polymer is chosen from homopolymers such as polylysine or polytyrosine; and copolymers such as maleic anhydride copolymers, N-vinylpyrrolidone copolymers, natural or synthetic polysaccharides and polynucleotides and amino acid copolymers such as enzymes.

8. Method according to Claim 7, **characterized in that** the polymer is chosen from maleic anhydride/methyl vinyl ether copolymer, N-vinylpyrrolidone/N-acryloxysuccinimide copolymer and poly-6-aminoglucose.

9. Method according to Claims 1 and/or 4, **characterized in that** the organic molecule is a hapten.

10. Method according to Claim 9, **characterized in that** the organic molecule is bound to a carrier molecule.

11. Method according to Claim 9, **characterized in that** the organic molecule is biotin and the carrier molecule is avidin.

12. Method according to Claim 4, **characterized in that** the organic molecule in the detection phase is the label.

13. Method according to Claims 1 and/or 4, **characterized in that** the tag is placed at the N- or C-terminal end of the protein material.

14. Method according to Claims 1 and/or 4, **characterized in that** the reactive function of the organic molecule is chosen from ester, acid, halocarbonyl, sulfhydryl, disulfide, epoxide, haloalkyl and aldehyde functions.

15. Method according to any one of Claims 1 to 14, **characterized in that** the protein material comprises an antigen and the biological material is an antibody which is specifically recognized by said antigen.

16. Method according to any one of Claims 1 to 14, **characterized in that** the protein material comprises an antibody and the biological material is an antigen which is specifically recognized by said antibody.

17. Capture phase for a target biological material, **characterized in that** it comprises an organic molecule containing at least one reactive function and at least one protein material capable of recognizing or of binding, specifically and directly or indirectly, to the target biological material, said protein material containing a specific site for covalent binding to the reactive function of the organic molecule, which consists of at least one tag comprising at least six contiguous lysine or lysine-based residues.

18. Detection phase for a target biological material, **characterized in that** it comprises an organic molecule containing at least one reactive function, at least one protein material capable of recognizing or of binding, specifically and directly or indirectly, to the target biological material, and a label, said protein material containing a specific site for covalent binding to the reactive function of the organic molecule, which consists of at least one tag comprising at least six contiguous lysine or lysine-based residues.

19. Capture or detection phase according to Claim 17 or 18, **characterized in that** the organic molecule is a particulate or linear polymer.

20. Capture or detection phase according to Claim 19, **characterized in that** the polymer is chosen from homopolymers such as polylysine or polytyrosine; and copolymers such as maleic anhydride copolymers, N-vinylpyrrolidone copolymers, natural or synthetic polysaccharides and polynucleotides and amino acid copolymers such as enzymes.

21. Capture or detection phase according to Claim 20, **characterized in that** the polymer is chosen from maleic anhydride/methyl vinyl ether copolymer, N-vinylpyrrolidone/N-acryloxysuccinimide copolymer, poly-6-aminoglucose, horseradish peroxidase (HRP) and alkaline phosphatase.

22. Capture phase according to Claim 21, **characterized in that** the organic molecule is a hapten, such as biotin.

23. Capture or detection phase according to Claim 17 or 18, **characterized in that** the tag is placed at the N- or C-terminal end of the protein material.

24. Capture or detection phase according to Claim 17 or 18, **characterized in that** the reactive function of the organic molecule is chosen from ester, acid, halocarbonyl, sulfhydryl, disulfide, epoxide, haloalkyl and aldehyde functions.

25. Detection phase according to Claim 18, **characterized in that** the organic molecule is the label.

26. Detection phase according to Claim 18 or 25, **characterized in that** the label is chosen from the group consisting of an enzyme, a protein, a peptide, an antibody, a hapten such as biotin or iminobiotin, a fluorescent compound such as rhodamine, a radioactive compound, a chemiluminescent compound, an electron-density component, a magnetic component and analogs.

27. Reagent for isolating a target biological material, comprising a capture phase according to Claim 17 and any one of Claims 19 to 24, and/or a detection phase according to any one of Claims 18 to 26.

28. Reagent according to Claim 27, **characterized in that** the capture phase is bound, directly or indirectly, to a solid support, by passive adsorption or by covalency.
